# EUROPEAN PATENT SPECIFICATION

(11) **EP 0 657 175 B1**
(45) Date of publication and mention of the grant of the patent: **02.03.2005**
(21) Application number: 94203576.7
(22) Date of filing: 08.12.1994
(51) Int. Cl.: A61K 39/385

(54) **Vaccine comprising human autologous epidermal growth factor and use thereof**
Impfstoff, der einen menschlichen autologenen epidermalen Wachstumsfaktor enthält und dessen Verwendung
Vaccin comprenant un facteur humain de croissance de l'épiderme autologue et son utilisation

(30) Priority: 09.12.1993 CU 11393
(43) Date of publication of application: 14.06.1995
(73) Proprietor: Centro de Inmunologia Molecular, Ciudad de la Habana 11600 (CU)
(72) Inventor: Dávila, Bienvenido Lage, Plaza, Ciudad de la Habana (CU); Marinello, Gisela González, Ciudad de la Habana (CU); Ramirez, Belinda Sánchez, Centro Habana, Ciudad de la Habana (CU); Peztana, Eduardo Suárez, 10 de Octubre, Ciudad de La Habana (CU); Delgado, Irene Beausoleil, Flores, Playa, Ciudad de La Habana (CU); Gandolf, Gilda Nunez, Regla, Ciudad de La Habana (CU)
(74) Representative: Smulders, Theodorus A.H.J., Ir.

(56) References cited:
- WO-A-88/00837
- WO-A-91/01146
- WO-A-91/09871
- PROCEEDINGS OF THE NATIONAL ACADEMY OF SCIENCES OF USA, vol. 84, January 1987, WASHINGTON US, pages 141-145, XP002010626 SARAH SPIEGEL ET AL.: "GANGLIOSIDES AS BIMODAL REGULATORS OF CELL GROWTH."

## Description

### FIELD OF THE INVENTION

This invention relates to the field of immunology, in particular to vaccine compositions able to produce an auto-immune reaction against autologous (self) Epidermal Growth Factor (EGF).

An important object of this invention is to obtain a vaccine composition for the active immunotherapy of EGF dependent malignant tumors, which can inhibit the proliferation of those tumors, and which therefore are useful for the treatment of malignant neoplasms and of other EGF related diseases. Thus, the invention is also related to the field of cancer therapy.

### DESCRIPTION OF THE PRIOR ART:

Epidermal Growth Factor, a polypeptide that stimulates epithelial cell proliferation, has been considered to be one of the growth factors involved in malignant transformations. Its action is mainly performed via its membrane receptors.

Epidermal Growth Factor (EGF) is a 53 amino acid polypeptide, its molecular weight is about 6045 D. It was isolated and purified for the first time from the murine submaxillary gland (Cohen S. J.Biol Chem (1962) 237,1.555), later a similar molecule was obtained from human urine (Cohen S. Human Epidermal Growth factor: Isolation and Chemical and Biological Properties PNAS USA 72,1975 1 317).

EGF is capable of stimulating the proliferation of epithelial and mesenchymal cells, both in vitro and in vivo (Cohen S., Carpenter G., PNAS USA 72, 1317, 1975) and EGF gives a specific stimulation in some breast cancer cell lines (Osborne C.K. et al. Can Res. 40,2.361 (1980). A role of the EGF in the differentiation process of the mammary gland, mainly for the development of the lobule alveolar system has been demonstrated(Tonelli C.J. Nature (1980) 285, 250-252).

This bio-regulating action is exerted via a membrane receptor (EGF-R), a 1,186 amino acid glycoprotein of about 170 kD, the gene of which has been cloned and sequenced. The intracellular domain of the receptor is associated with an activity of Tyrosine specific protein kinase which shows a structural homology to the oncogene product v-erb-B showing the relation to the malignant transformation process (Heldin C.H. Cell 37, 9-20 (1984)).

EGF and its receptor constitute a molecular complex of high specificity and the interaction between them develops important mechanisms of cell growth regulation.

High levels of EGF-R have been detected in malignant tumors of epithelial origin, such as breast, bladder, ovarian, vulva, colonic, pulmonary, brain and oesophagus cancers. The role played by EGF and its receptor in regulating tumor growth is unknown, but there are suggestions that the EGF-R expression in tumor cells provides a mechanism for autocrine growth stimulation which leads to uncontrolled proliferation (Schlessinger J., Schreiber A.B., Levi A., Liberman T., Yarden Y. Crit. Rev. Biochem. 1983, 14 (2) 93-111).

The presence of EGF-R in tumor cells has proven to be an indication of a poor prognosis in human breast cancer. Approximately 40% of the breast tumors show specific binding sites of high affinity for EGF, there is also an inverse correlation with the presence of oestrogen receptor indicating EGF-R as a dedifferentiation marker or an indicator of the potential capacity of proliferation of the malignant cells. (Perez R., Pascual M.R., Macias A., Lage A., Breast Cancer Research and Treatment 4, 189-193,1984).

It also has been reported that the EGF-R expression is higher in regional ganglional metastases than in the primary breast carcinomas (Sainsbury J.R.,et al..(1985): Lancet 1(8.425), 364-366), and that the expression of the receptor is different in the different histologic subtypes of breast carcinomas cells, which also makes their presence a signal of bad prognosis( Macias A., et al. (1986); Anticancer Res.6:849-852).

Previous studies performed in the Ehrlich Ascitic Tumor (EAT) model in Balb-C mouse , demonstrated the in vivo inhibitory effect of EGF .(Lombardero J., et al. Neoplasma 33, 4 (1987) suggesting the possibility of considering this molecule as a biological response modifier.

It has been previously reported the presence of an EGF precursor molecule in the cell membrane of EGF dependent tumors. The authors of the present invention have considered this an important fact to consider this molecule as a target for the action of auto-antibodies.

The results obtained in different studies, have prompted the consideration of the EGF\EGF-R system as a possible target for therapeutic actions.

Passive immunotherapy using monoclonal antibodies against the EGF-R, has been the object of multiple investigations, which have demonstrated that the specific recognition by the the antibody of the receptor inhibits the EGF binding, with an inhibitory effect on the mitogenic stimulation of malignant cells (SATO J.D.,et al. Methods in Enzymology, vol. 146 pp 63-81.(1987), however, these antibodies which are of murine origin will usually produce a human anti mouse antibody response (HAMA).

Until the present invention no active immunotherapy against EGF dependent tumors capable of inhibiting proliferation has been proposed, since the art consistently reports that "self" molecules will not induce any immune reaction because the host has been educated to be tolerant to self.

The present invention provides a vaccine composition containing human autologous EGF coupled to a carrier protein, which complex will inhibit the EGF dependent tumors' growth, through an autoimmune effect, without the collateral effects of the introduction of a heterologous protein in the human body.

This vaccine composition can be used in the treatment of EGF dependent tumors or any malignant disease associated to EGF.

It will be understood that in this specification EGF is to be read as including any fragment and/or derivative of EGF which has similar immunological properties and/or effects as the original molecule. Derivatives include, but are not limited to, conventional aminoacid substitutions, site-directed replacement of aminoacids for enhanced stability and/or activity, chemical modifications and the like.

### DETAILED DESCRIPTION OF THE INVENTION:

### I. OBTAINING AN IMMUNOGENIC PREPARATION:

Two preparations were obtained. The first is murine EGF (mu-EGF) coupled to a protein carrier and the second is human recombinant EGF (hu-re-EGF) (National Medicament Register Office from Cuba, HEBERMIN, No.1266), also coupled to a carrier protein.

The preparation containing hu-rec-EGF coupled to a carrier protein was obtained to be used only in non human primates and in humans. A proper adjuvant is applied.

The mu-EGF conjugated to a protein carrier is used in the studies performed in mice as a model to determine the immunogenicity and the antitumoral effect of a preparation containing an autologous EGF molecule.

Immunogenicity studies were performed in primates with the conjugate hu-EGF-carrier protein carrier since the hu-EGF is very similar to the primate EGF though it is recognized as a self molecule. These results allowed to demonstrate the immunogenic response an autologous molecule can elicit.

To obtain the preparations a solution of murine or hu-rec-EGF in PBS/MgCl₂ 10 mM, is mixed with a solution of the carrier protein in the same solvent, in a ratio of between 1 and 5 moles of EGF per mol of protein.

Afterwards glutaraldehyde 0.5%, is added to obtain a final concentration between 0.1% and 0.05%.

The mixture is incubated between 1 and 3 hours at room temperature and subsequently dialyzed in PBS/MgC12 10 mM with, at least, 3 changes of dialysis solution.

### CONJUGATE CHARACTERIZATION:

The test of the conjugation efficiency and of the maintenance of the antigenicity is performed through an ELISA assay.

ELISA plates of activated PVC (NUNC) were coated with 50 µl of an antiserum against the carrier protein utilized, in a concentration between 1 and 10 µg/ml. In the case of cholera toxin chaim B (CTB) as carrier, the plates were coated with the ganglioside GMI.

Subsequently 3 washes with PBS/Tween (registered trademark) were carried out and then the plates were blocked with a solution of BSA between 0.5 and 1% in PBS/Tween, incubated them during a period of 30 minutes to 1 hour at 37°C. Dilutions between 0.1 and 0.001 mg/ml of the conjugates to be assayed were added to the plates, 50 µl/well, and incubated for 1 to 2 hours at 37°C.

In the next step a mouse anti hu-rec-EGF antiserum was added in a dilution between 1:500 and 1:1000, 50 µl/well, and incubated for between 30 minutes and 1 hour at 37°C.

As the last step, the plates were incubated with an anti-mouse-alkaline phosphatase antiserum, in a dilution between 1:500 and 1:1000, 50 µl/well, for 30 minutes to 1 hour at 37°C.

Reaction color was developed with p-nitrophenylphosphate, at a concentration of 1 mg/ml in diethanolamine, 50 µl/well, incubated for 30 minutes at 37°C. Optical density was measured at 405 nm in an ELISA plate reader.

The results demonstrated the activity of the molecule and the efficiency of the conjugation, because the conjugate maintains its recognition site for the molecule coating the plates, which specifically recognizes the carrier protein and, at the same time can be recognized by an anti EGF antiserum.

### II.-CHARACTERIZATION OF EFFECTS PRODUCED BY THE PREPARATION CONTAINING mu-EGF. PRE-CLINICAL STUDIES.

### IIa) ENDOGENOUS EGF IMMUNOGENICITY: INDUCTION OF AUTO-IMMUNITY IN MICE.

In order to demonstrate the capacity of the immunogenic preparation containing mu-EGF obtained through the technique described in item I of inducing autoimmunity against the endogen EGF, a test was performed in Balb/C mice.

Groups of animals were inoculated with different doses in the range of 50 to 100 µg of mu-EGF conjugated to the carrier protein per animal per week during 4 to 6 weeks.

The first week the immunogenic preparation was prepared in a ratio of 1:1 with complete Freund's adjuvant, all the following doses were prepared with incomplete Freund's adjuvant.

The same procedure was performed in a control group, but only adjuvant was administered to the animals. One week after the last immunization, blood was extracted to the animals, the serum separated from the remainder of the blood and the titer of antibodies against mu-EGF was determined by an ELISA technique.

### II b) IMMUNOGENICITY OF hu-rec-EGF:

In order to demonstrate the immunogenicity of hu-rec-EGF in mice and to show that the antibodies against hu-rec-EGF recognize the mu-EGF, the experiment was performed in Balb/C mice.

Groups of animals are inoculated with different doses in the range of 50 to 100 µg of hu-rec EGF-protein per animal per week during 4 to 6 weeks.

The first week the immunogenic preparation was prepared in a ratio of 1:1 with complete Freund's adjuvant, all the following doses were prepared with incomplete Freund's adjuvant.

The same procedure is performed in a control group, but only adjuvant is administered to the animals.

One week after the last immunization, blood was extracted from the animals, the serum was separated from the rest of the blood and the titer of antibodies against mu-EGF was determined by an ELISA technique.

### IIc) ANTITUMOR ACTIVITY:

The main objective of this experimental procedure is to determine whether the immune response obtained against the autologous EGF is able to elicit any antitumor effect in EGF dependent tumors.

The animals with higher antibody titer, determined according to the technique described previously, were inoculated with Ehrlich Ascitic Tumor (EAT) in cellular concentrations between 0.2 to 2 million cells per animal. The control group was treated in the same manner.

The animals were observed for grafting as well as for survival.

### III. CHARACTERIZATION OF THE IMMUNE RESPONSE:

### IIIa) ISOTYPE OF THE ANTIBODY RESPONSE

In order to determine whether the autoimmune response obtained upon the immunization of mice with the autologous EGF is a response producing antibodies of the isotype IgM or IgG, an ELISA assay was performed testing the sera of immunized animals with EGF according to the techniques described in items II a) and b). In the case of IgM characterization an antiserum against this molecule was incubated with the samples. IgG characterization is performed with an antiserum against IgG.

### IIIb) CHARACTERIZATION OF THE MEMORY OF THE IMMUNE RESPONSE

The development of a product to be used as a vaccine in an active therapy requires the determination of its capability of inducing an immunological memory and the determination of the duration of said memory if it is induced.

This information allows the possibility of a correct design of the immunization schemes that can be implemented with the product.

Groups of mice are immunized with one dose between 50 and 100 µg of hu-rec-EGF per animal in complete Freund's adjuvant in a proportion 1:1.

The kinetic of antibody production against mu-EGF was studied in different groups of animals. This study was performed after the first immunization and after the re-immunizations when the titer is declining. The antibody levels are determined using an ELISA technique.

### IV. IMMUNOGENICITY STUDIES IN NON HUMAN PRIMATES.

The criteria of immunogenicity of the immunogenic preparation are based on results obtained in non human primates because these are the species that are the closest to human.

A group of primates are immunized with the immunogenic preparation containing hu-rec-EGF in a dose of 500 µg (conjugated with tetanic toxoid) and together with the adjuvant. After the last immunization a sample of blood was extracted and antibody titers against hu-EGF were determined.

### EXPERIMENTS

### EXAMPLE 1.

### STUDY OF THE PRESENCE OF AN EGF PRECURSOR MOLECULE IN THE CELL MEMBRANE OF EGF DEPENDENT TUMORS.

This study was performed through a Western Blotting technique. Samples of 5 ductal carcinoma of the breast in different stages, one head and neck tumor, four samples of fibrocystic dysplasia and five normal samples obtained as controls were studied.

Cell membranes were obtained from the samples through the procedure described elsewhere (Grimaux M.,Rev Neurol. 1988, 144: 101-103).

Electrophoresis was performed at 250 v, 10 mA, 3. OW, 15°C and 150 Vh. Molecular weight standards were used in the range of 14 300 D (Lysozyme) to 340 000 D (alpha 2 macroglobulin).

Proteins separated during electrophoresis were transferred to a nitrocellulose membrane of 0.45 µm in a Phast system equipment in a buffer transfer solution. After the transference the membrane was blocked overnight with 10% skimmed milk with constant stirring.

After three washes in buffer solution a mouse monoclonal antibody recognizing human EGF was added and incubated during one hour.

After three washes a biotynilated anti mouse antibody was added and incubated during one hour. Peroxidase streptavidine conjugate was added and reaction developed with diaminobenzidine and hydrogen peroxide after one hour of incubation.

The results obtained demonstrated that the samples studied corresponding to normal tissues did not show any band in the zone of high molecular weight according to the standards. However, the samples corresponding to breast pathology (dysplasia and carcinomas) showed a diffuse banding in the high molecular weight zone. This is an experimental evidence of the presence of a high MW EGF precursor in the tumor membranes.

### EXAMPLE 2

### OBTAINING THE mu-EGF/CTB CONJUGATE:

One ml of mu-EGF in PBS/MgCl₂ 10 mM at a concentration of 1mg/ml, was mixed with 2 ml of a solution of CTB in the same solvent at a ratio of 1 mol of mu EGF per mol of CTB. Glutaraldehyde (3 ml, 0.5%) was added to obtain a final concentration of 0.05%.

Incubation was performed during 1 hour at room temperature and subsequently dialyzed in PBS/MgCl₂ 10 mM with, at least, 3 changes of the dialysis solution.

### EXAMPLE 3

### mu-EGF/CTB CONJUGATED CHARACTERIZATION:

ELISA assay for conjugate test: PVC activated ELISA plates (NUNC) were coated with 50 µl of the GM1 ganglioside (recognizing the CTB molecule) in a concentration of 4 µg/ml in methanol, which was left to dry off in the flow during 1 hour.

Subsequently 3 washes with PBS/Tween were carried out and then the plates were blocked with a solution of BSA 1% in PBS/Tween and incubated during 30 minutes at 37°C.

Conjugated dilutions between 0.1 and 0.001 mg/ml were added to the plates at 50 µl/well and incubated during 1 hour at 37°C.

Next a mouse anti-mu-EGF antiserum in a 1:1000 dilution, 50 µl/well was added and incubated for 1 hour at 37°C.

Then, the plates were incubated with anti mouse antiserum alkaline phosphatase conjugate (dilution 1:1000), 50 µl/well for 1 hour at 37°C. The color was developed with p-nitrophenylphosphate at a concentration of 1 mg/ml in diethanolamine, 50 µl/well, incubated for 30 minutes at 37°C, optical density was measured at 405 nm.

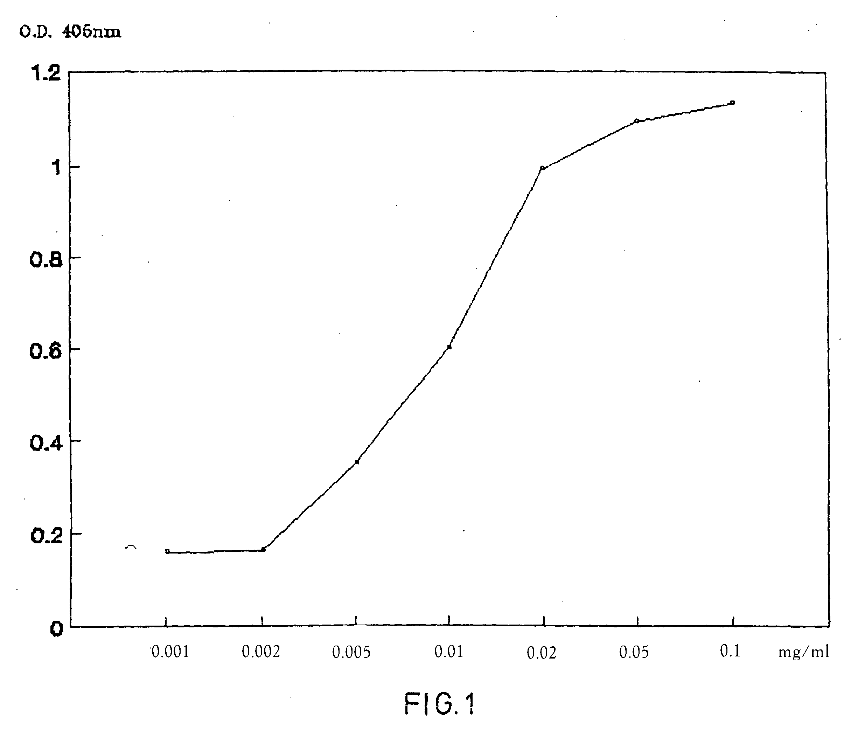

The results demonstrated a direct relationship between the concentration of the conjugated and the absorbance values. This demonstrates the activity of the conjugated and the efficiency of the conjugation, since, the molecule maintains the recognition for the GM1 ganglioside (identifies CTB) and, at the same time, is recognized by an anti mu-EGF antiserum (Figure 1)

### EXAMPLE 4.

### IMMUNOGENICITY OF AUTOLOGOUS EGF: INDUCTION OF AUTO-IMMUNITY IN MICE:

In order to demonstrate that the immunogenic preparation containing autologous EGF is capable of inducing autoimmunity, the experimentation was performed in Balb/c mice.

Groups of animals were inoculated with a dose of 50 µg of conjugated mu-EGF per animal subcutaneously weekly during 4 to 6 weeks.

The first week the immunogenic preparation was prepared in a proportion 1:1 with complete Freund's adjuvant, all the following doses were prepared with incomplete Freund's adjuvant.

The same procedure was performed in a control group, but only adjuvant was administered to the animals. One week after the last immunization, blood was extracted from the animals, the serum obtained and the titer of antibodies against mu-EGF was determined by an ELISA technique.

Costar plates were coated with mu-EGF at a concentration of 10 µg/ml in carbonate bicarbonate buffer (pH 9.6), and incubated overnight. After the plates were washed the samples were added in different dilutions. Incubation took place during one hour. Alkaline phosphatase anti-mouse antibody conjugate was added and incubated during one hour after which color was developed and optical density measured at 405 nm in an ELISA reader.

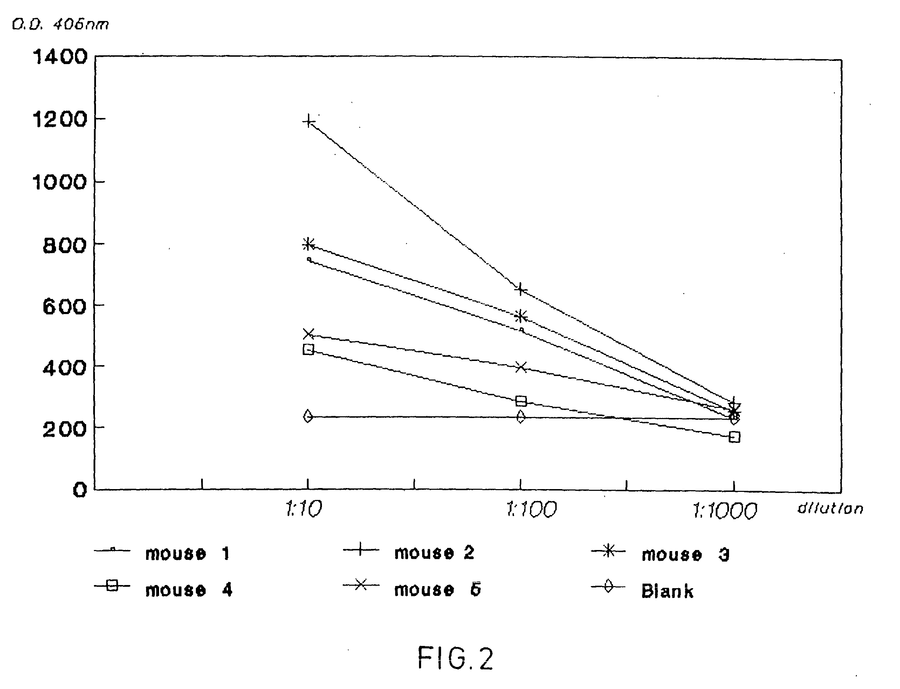

All the animals immunized with the mu-EGF-CTB preparation developed antibody titer against the mu-EGF up to 1:1000 dilution. The control group did not show any antibody titer (Figure 2)

### EXAMPLE 5

### IMMUNOGENICITY OF hu-rec EGF: INDUCTION OF AUTO-IMMUNITY IN MICE:

In order to demonstrate that the immunogenic preparation containing hum-rec EGF was capable of producing antibody titer against mu-EGF, the experimentation was performed in Balb/c mice.

Groups of animals were inoculated with a dose of 50 µg of hum-rec-EGF per animal subcutaneously, weekly during 4 to 6 weeks.

The first week the immunogenic preparation was prepared in a proportion 1:1 with complete Freund's adjuvant, all the following doses were prepared with incomplete Freund's adjuvant.

The same procedure was performed in a control group, but only adjuvant was administered to the animals.

One week after the last immunization, blood was extracted from the animals, the serum obtained and the titer of antibodies against mu-EGF was determined by an ELISA technique.

Costar plates were coated with mu-EGF at a concentration of 10 µg/ml in carbonate bicarbonate buffer (pH 9.6), and incubated overnight. After the plates were washed the samples were added in different dilutions. Incubation took place during one hour. The alkaline phosphatase antimouse antibody conjugate was added and incubated during one hour after which color was developed and optical density measured at 405 nm in an ELISA reader.

All the animals immunized with the hum-rec EGF preparation developed antibody titer against the mu-EGF up to 1:20000 dilution.

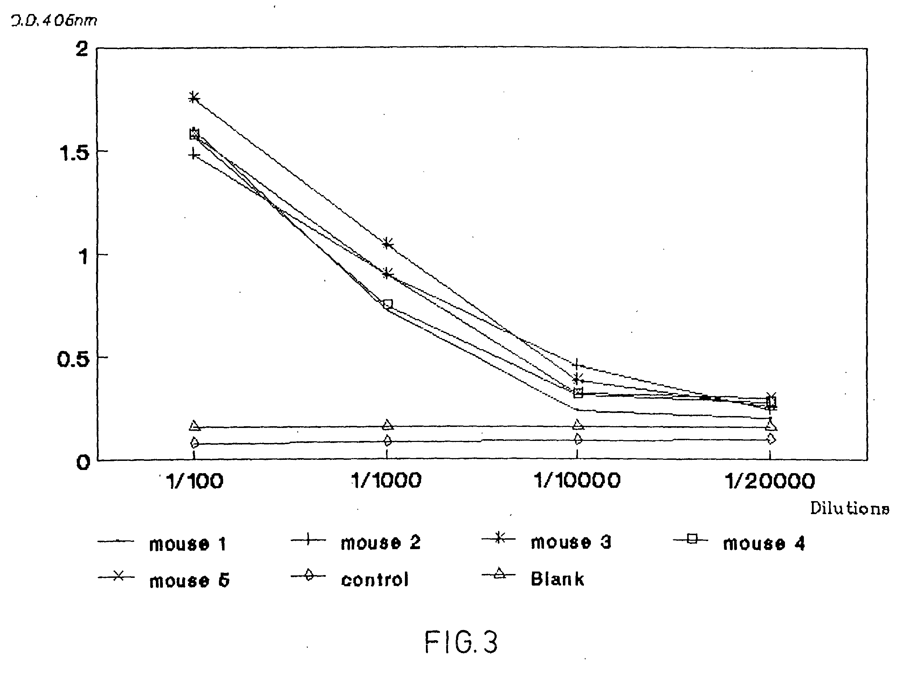

The control group did not show any antibody titer.(Figure 3).

### EXAMPLE 6

### IMMUNOGENICITY OF hu-rec EGF IN A PREPARATION WITH ALUMINIUM HYDROXIDE.

In order to demonstrate that the immunogenic preparation containing hum-rec EGF and Aluminium Hydroxide as adjuvant was capable of producing antibody titer against mu-EGF, the experimentation was performed in Balb/c mice.

Groups of animals were inoculated with a dose of 50 µg of hum-rec-EGF (with Aluminium Hydroxide as adjuvant) per animal subcutaneously, weekly during 4 to 6 weeks.

The same procedure was performed in a control group, but only adjuvant was administered to the animals. One week after the last immunization, blood was extracted from the animals, the serum obtained and the titer of antibodies against mu-EGF was determined by an ELISA technique.

Costar plates were coated with mu-EGF at a concentration of 10 µg/ml in carbonate bicarbonate buffer (pH 9.6), and incubated overnight. After the plates were washed the samples were added in different dilutions. Incubation took place during one hour. The alkaline phosphatase antimouse antibody conjugate was added and incubated during one hour, after which color was developed and optical density measured at 405 nm in an ELISA reader.

All the animals immunized with the hum-rec EGF/Al OH preparation developed antibody titer against the mu-EGF up to 1:4000 dilution.

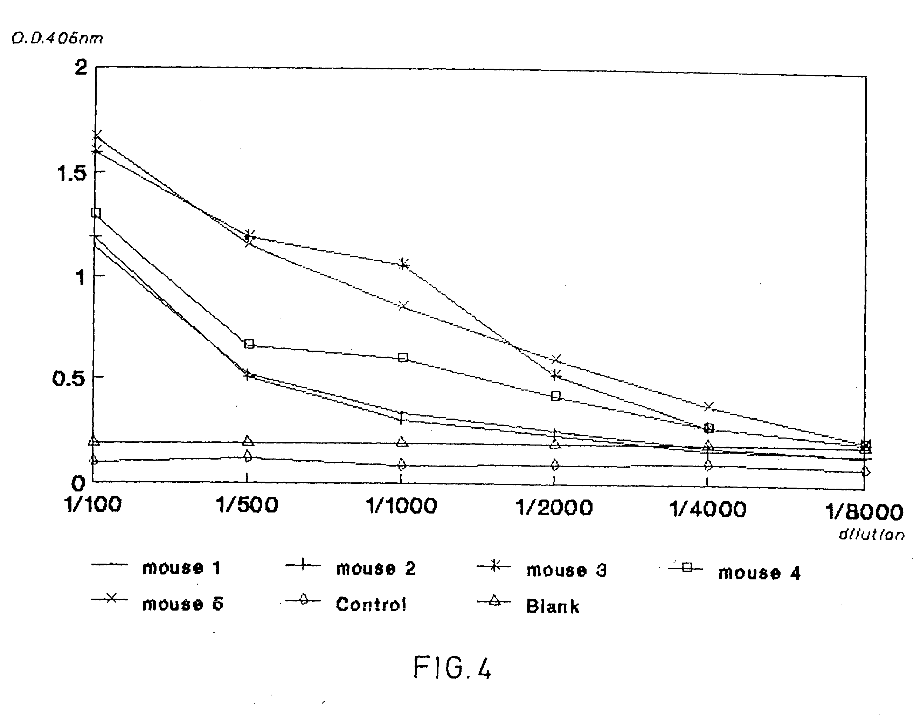

The control group did not show any antibody titer (Figure 4).

### EXAMPLE 7

### ANTI-TUMOR ACTIVITY:

The main objective of this experimental procedure was to determine whether the immune response obtained against the autologous EGF was able to elicit any antitumor effect in EGF dependent tumors.

The animals with higher antibody titer, determined according to the technique described previously in the example 5, were inoculated with Ehrlich Ascitic Tumor (EAT) in cellular concentrations of 2 million cells EAT per animal. The control group (nonimmunized mice) was treated in the same manner.

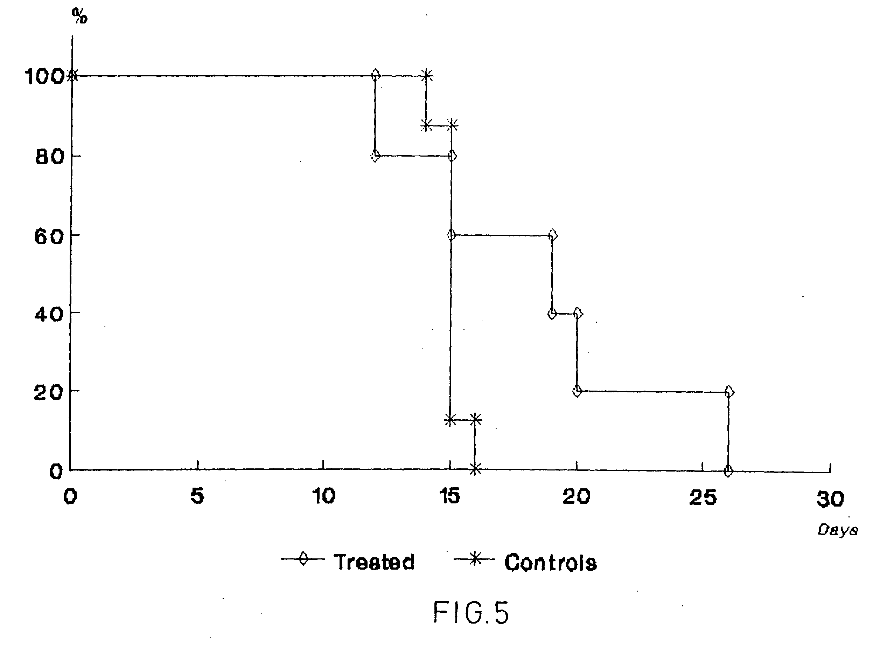

The animals were observed for grafting as well as for survival. Survival curves of treated and control animals are shown in Figure 5.

The Increase in Life Span Index was 22.5% showing an increase in survival for the treated animals in relation to the control statistically significant according to the Mantel Haenszel and Wilcoxon tests.

### EXAMPLE 8

### ASSOCIATION BETWEEN ANTIBODY TITER AGAINST mu EGF AND ¹²⁵I EGF BIODISTRIBUTION.

This experiment was performed to demonstrate that there is a different biodistribution of ¹²⁵I EGF in animals with antibody titer against mu- EGF in relation to animals that did not have antibody titer against mu-EGF.

An experiment with 4 groups of mice was performed for this purpose:
Group 1: 30 mice with antibody titer against mu-EGF.
Group 2: 30 mice without antibody titer against mu-EGF.
Group 3: 30 mice with antibody titer against mu-EGF grafted with EAT.
Group 4: 30 mice without antibody titer against mu-EGF grafted with EAT.

Samples from group 1 and 2 were taken from blood, lung, kidneys, liver and skin at the following times: 2, 5, 8, 11, 15, 20, 30, 60, 120 and 150 minutes and 3 animals were sacrificed at every corresponding time, counting the radioactivity in the organs extracted.

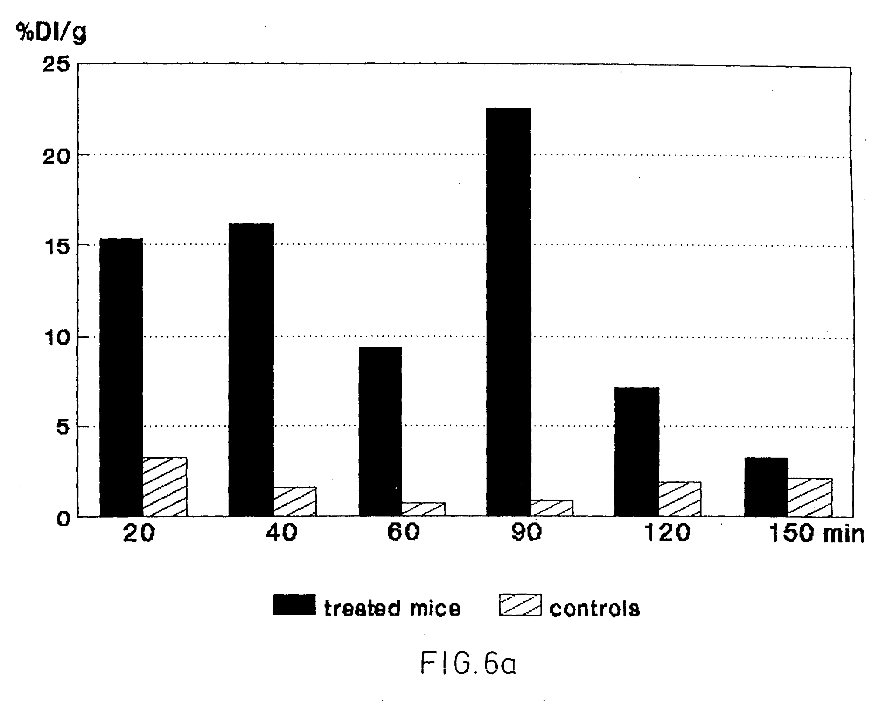

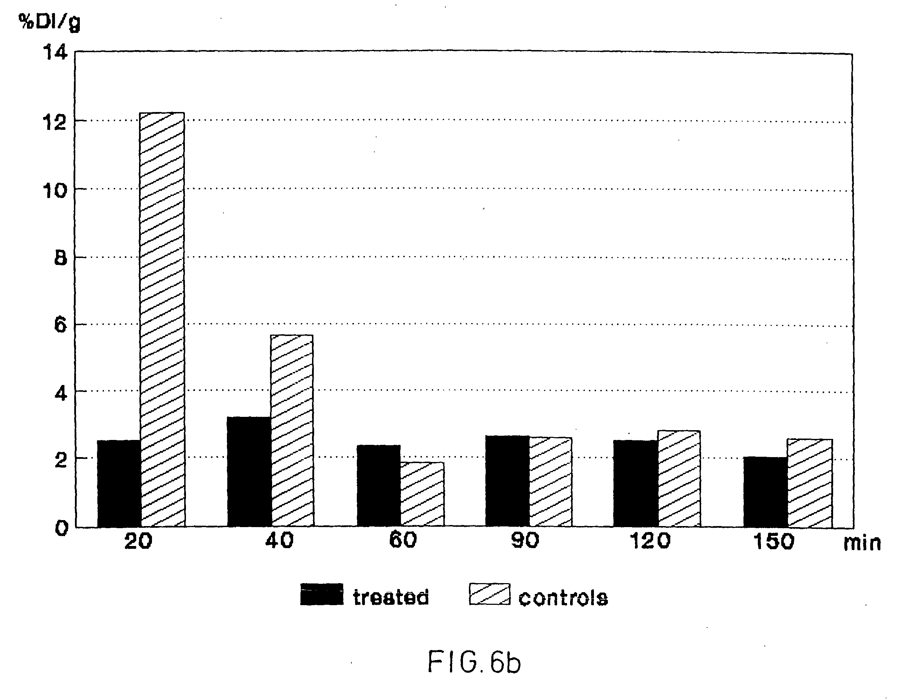

The results obtained have shown a difference in the accumulation of I-125 EGF in time mainly kidney and liver (Figure 6 a,b), indicated that the presence of antibodies against EGF do alter the biodistribution of this molecule.

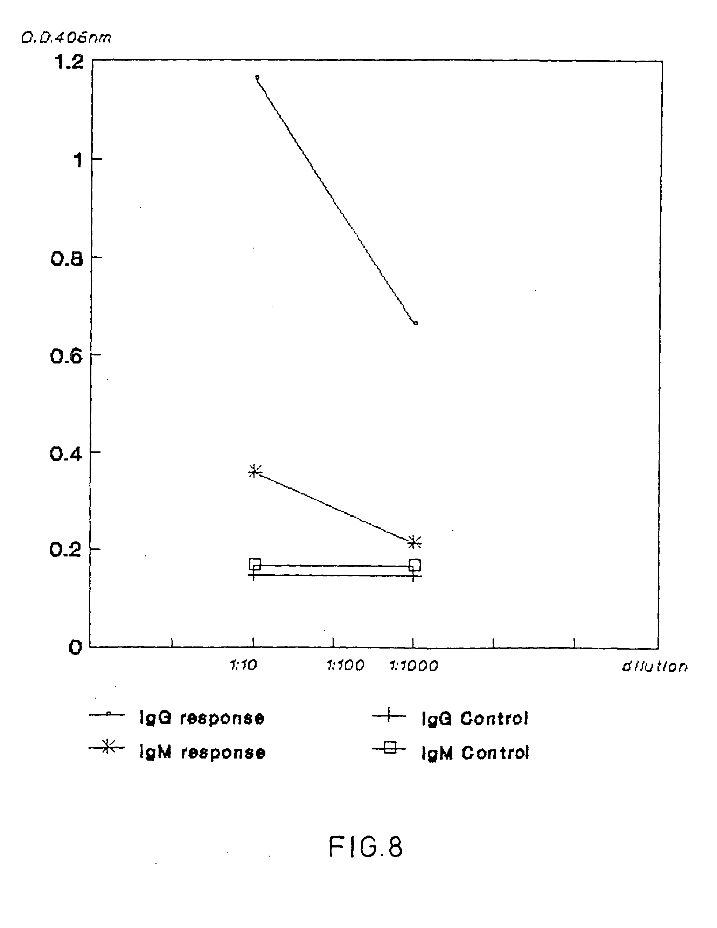

The results obtained have also shown that there is a difference between the animals with or with without antibody titer (Figure 8,a,b,c,d) indicating that the animals with antibody titer can produce immunocomplexes with the circulating EGF showing a different depuration mechanism of the labelled EGF.

Samples from group 3 and 4 were taken from blood, lung, kidneys, liver, skin and from the ascitic fluid at the following times: 2, 5, 8, 11, 15, 20, 30, 60, 120 and 150 minutes and 3 animals were sacrificed at every corresponding time, and the radioactivity counted in the organs extracted.

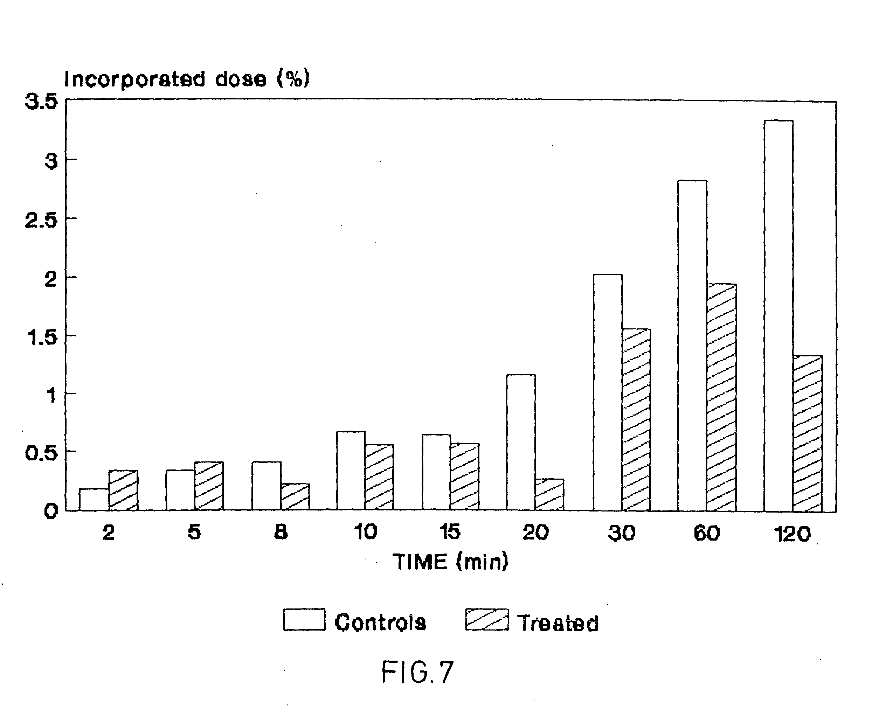

It could be appreciated less accumulation of the labelled EGF in the ascitic fluid of animals with antibody titer than in the animals without antibody titer (Figure 7), indicating a more rapid depuration of the EGF present in the ascitic fluid in these animals and/or a limitation in EGF access to the ascites.

### EXAMPLE 9:

### IMMUNE RESPONSE CHARACTERIZATION: ISOTYPE OBTAINED AGAINST AUTOLOGOUS EGF.

In order to know whether the autoimmune response obtained upon the immunization of mice with the autologous EGF was a response producing antibodies of the isotype IgM or IgG, an ELISA assay was performed, in which the plates were coated with EGF to concentration of 10 µg/ml, 50 µg/well, and incubated for 1 hour at 37°C.

Subsequently, dilutions between 1:10 and 1:1000 of the serums of animals immunized with mu EGF-CTB according to Example 5 were applied, 50 µg/well and were incubated for 1 hour at 37°C.

Parallel design of plates were performed to measured IgG or IgM response with the corresponding antiserum (anti-IgG or anti-IgM respectively).

Color in the plates was developed with p-nitrophenyl phosphate, at a concentration of 1 mg/ml in diethanolamine, incubating during 30 minutes at 37°C and values of optical density at 405 nm were read.

IgG response was obtained in all treated animals (Figure 8)

### EXAMPLE 10

### CHARACTERIZATION OF THE MEMORY OF THE IMMUNE RESPONSE AGAINST AUTOLOGOUS EGF.

Two groups of 10 mice were studied with a single immunization of 50 µg hu-re-EGF in complete Freund's adjuvant.
**Group I**.-The kinetics of antibody production against mu EGF was studied in this group of animals. Every 4 days blood samples were extracted. The antibody levels were determined by an ELISA technique.
**Group II**. - Was conformed with the animals with declining antibody titers from Group I and immunized again. Every 2 days blood samples were extracted. The antibody levels were determined by an ELISA technique.

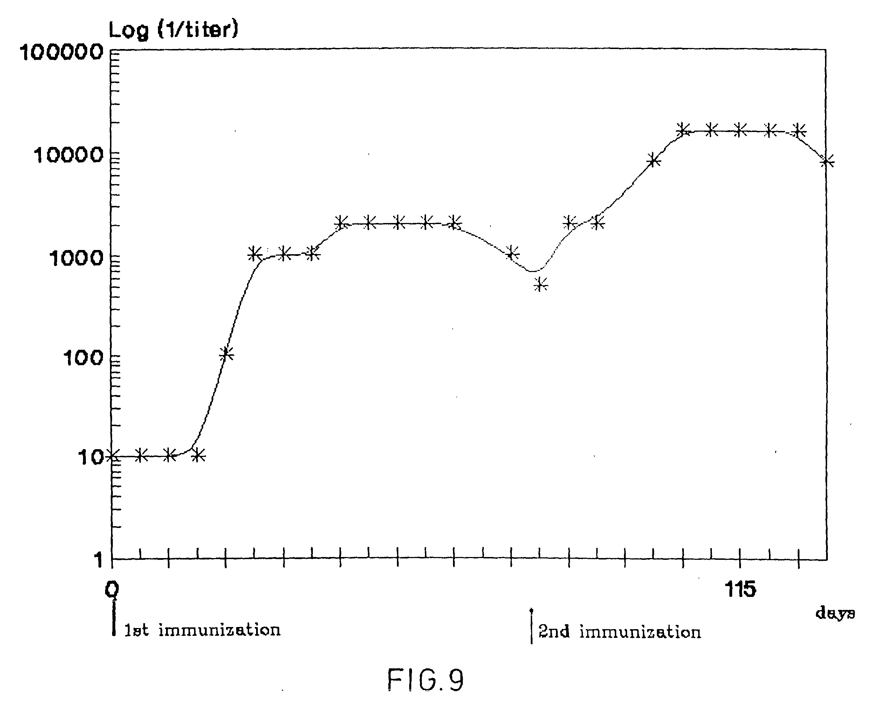

Results have shown a memory response when the animals were immunized again with the preparation (Figure 9).

### EXAMPLE 11.

### OBTENTION OF THE IMMUNOGENIC PREPARATION: hu-rec EGF TOXOID TETANIC

A solution of hu-EGF in PBS/MgCl₂ 10 mM at a concentration of 1.4 mg/ml, was mixed with 2 ml of a solution of TT in the same solvent at a concentration of 4 mg/ml. Glutaraldehyde (3 ml, 0.5%),was added to obtain a final concentration of 0.05%.

Incubation was performed during 1 hour at room temperature and subsequently dialysed in PBS/MgCl₂ 10 mM with, at least, 3 changes of the dialysis solution.

### EXAMPLE 13

### CONJUGATE CHARACTERIZATION: hu-rec EGF toxoid tetanic ELISA assay for conjugate test:

Costar plates (High Binding) were coated with 50 µl of an antiserum anti TT obtained in sheep, in a concentration of 10 µg/ml and incubating it overnight.

Subsequently 3 washes with PBS/Tween were carried out and then the plates were blocked with a solution of BSA 1% in PBS/Tween, and incubated during 30 minutes at 37°C.

Conjugate dilutions between 0.1 and 0.001 mg/ml were added to the plates at 50 µl/well, and incubated during 1 hour at 37°C.

Next a mouse anti-hu-EGF antiserum in a 1:1000 dilution, 50 µl/well was added, and incubated for 1 hour at 37°C.

Then, the plates were incubated with anti mouse antiserum alkaline phosphatase conjugate (dilution 1:1000), 50 µl/well for 1 hour at 37°C. The color was developed with p-nitrophenylphosphate at a concentration of 1 mg/ml in diethanolamine, 50 µl/well, incubated for 30 minutes at 37°C, and optical density was measured at 405 nm.

The results demonstrated a direct relationship between the concentration of the conjugated and the absorbance values.

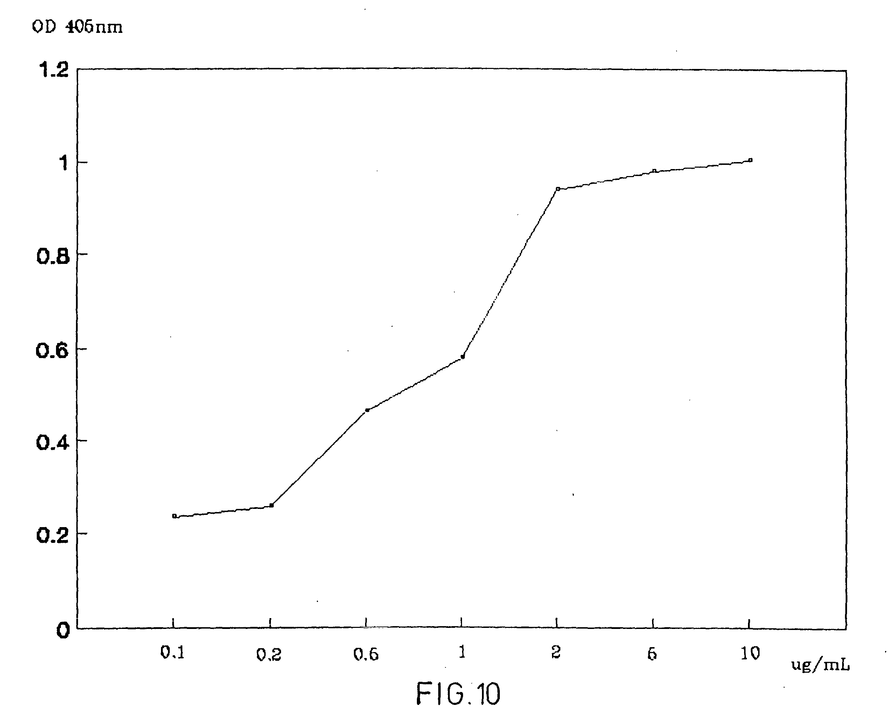

This demonstrates the activity of the conjugate and the efficiency of the conjugation, since, the molecule maintains the recognition for anti serum anti TT and at the same time, is recognized by an anti mu EGF antiserum (Figure 10).

### EXAMPLE 13

### STUDY OF THE IMMUNOGENICITY OF hu-EGF COUPLED TO A CARRIER PROTEIN (TT) IN NON HUMAN PRIMATES.

The study was performed with 4 Rhesus monkeys, being submitted to a clinical veterinary examination including:
Physical examination
Thorax X rays
Blood tests.

These animals were immunized with a hu-EGF coupled to TT, according to Example 10.

Immunization was performed subcutaneously in weeks 1, 2, 3, 4, 6 and 12th. Complete Freund's adjuvant was used in the first immunization and incomplete Freund's adjuvant in all others.

Blood was extracted from the animals and antibody titers were determined through an ELISA.

Costar plates were coated with hu-EGF at a concentration of 10 µg/ml in carbonate bicarbonate buffer (pH 9.6), and incubated overnight. After the plates were washed the samples were added in different dilutions Incubation took place during one hour. The alkaline phosphatase antihuman antibody conjugate was added and incubated during one hour after which color was developed and optical density measured at 405 nm in an ELISA reader.

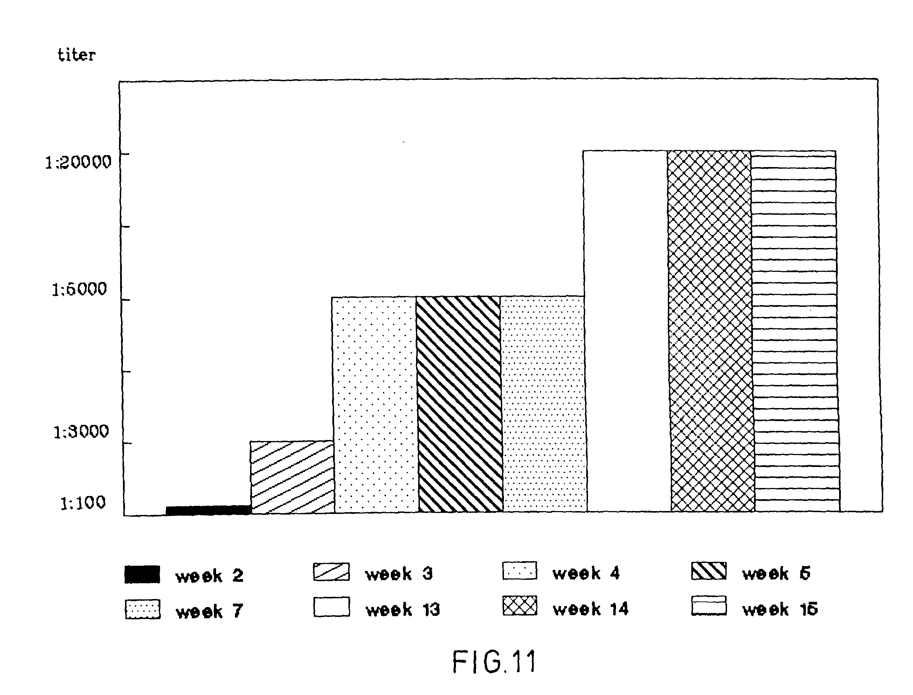

All the animals immunized with the hu-EGF-TT preparation developed antibody titer against the hu-EGF up to 1:20000 dilution (Figure 11)

### BRIEF DESCRIPTION OF THE DRAWINGS

**Figure 1**: ELISA assay for determination of conjugation efficiency between CTB and hu rec EGF.
   X axis: serial dilutions of the conjugate (from 0.1 mg/ml to 0.001 mg/ml)
   Y axis : optical density at 405 nm , measured in an ELISA plates reader.
**Figure 2**: ELISA assay for the determination of antibody titer against the mu EGF in 5 mice immunized with the conjugated mu EGF - CTB.
   X axis : Antiserum dilutions (1:10, 1:100, 1:1000)
   Y axis : optical density at 405 nm , measured in an ELISA plates reader.
   The curves represent the titer of 5 tested animals, compared with the same animals before immunization.
**Figure 3**: ELISA assay for the determination of antibody titer against mu-EGF, in 5 mice immunized with hu-rec-EGF.
   x axis: sera dilutions 1:100, 1:1000, 1:10000, 1:20000.
   y axis: optical density at 405 nm
**Figure 4**: ELISA assay for the determination of antibody titer against mu-EGF, in 5 mice immunized with hu-rec-EGF in Aluminium Hydroxide as adjuvant
   x axis: sera dilutions 1:100, 1:500, 1:1000, 1:2000, 1:4000, 1:8000.
   y axis: optical density at 405 nm
**Figure 5** : Survival of animals immunized with mu EGF-CTB and subsequently inoculated with Ehrlich Ascites Tumour, in comparison with control animals (nonimmunized) and inoculated with same tumor.
**Figure 6-a**: Accumulation of 125-I EGF in liver of mice immunized with hu-rec-EGF in relation to nonimmunized controls.
**Figure 6-b**: Accumulation of 125-I EGF in kidneys of mice immunized with hu-rec-EGF in relation to nonimmunized mice.
**Figure 7**: Accumulation of mu-EGF I¹²⁵, in ascitic fluid of animals grafted with EAT, previously immunized with hu-rec-EGF.
**Figure 8**: ELISA assay for determination of IgG or IgM of the immune response in animals immunized with mu EGF-CTB.
**Figure 9**: Antibody response kinetic (IgG) against mu EGF, in animals immunized against hu-rec-EGF (Memory).
   x axis: time (days).
   y axis: Inverse logarithm of the antibody titer.(mean value).
**Figure 10**: ELISA assay for the determination of the efficiency of conjugation with Tetanic toxoid and hu-rec-EGF.
   x axis: dilutions of conjugate.
   y axis: optical density at 405 nm
**Figure 11**: antibody titer against hu-rec-EGF in non human primates immunized with hu-rec-EGF, coupled to tetanic toxoid.

## Claims

1. A vaccine composition for producing an autoimmune response against autologous Epidermal Growth Factor comprising human autologous Epidermal Growth Factor conjugated to a carrier protein and further comprising an adjuvant, said human autologous Epidermal Growth Factor being an antigen for eliciting said autoimmune response.

2. A vaccine composition according to claim 1, **characterized in that** it contains recombinant human Epidermal Growth Factor.

3. A vaccine composition according to claim 1, **characterized in that** it contains the cholera toxin B chain as carrier protein.

4. A vaccine composition according to claim 1, **characterized in that** it contains tetanic toxoid as carrier protein.

5. A vaccine composition according to claim 1, **characterized in that** it contains a monoclonal antibody as carrier protein.

6. A vaccine composition according to claim 1, **characterized in that** it contains Neisseria meningitidis outer membrane protein as carrier protein.

7. A vaccine composition according to claim 1, **characterized in that** it contains aluminium hydroxide as adjuvant.

8. A vaccine composition according to any one of claims 1-7 for the treatment of an Epidermal Growth Factor-dependent malignant disease.

9. Use of autologous Epidermal Growth Factor of a mammal in the preparation of a vaccine according to any one of claims 1-7 for the treatment of an Epidermal Growth Factor-dependent malignant disease.

10. Use according to claim 9, **characterized in that** the vaccine is for the treatment of an Epidermal Growth Factor-dependent malignant disease in humans.

## Patentansprüche

1. Impfstoffzusammensetzung, um eine Autoimmunreaktion auf einen autologen epidermalen Wachstumsfaktor hervorzurufen, umfassend einen humanen autologen epidermalen Wachstumsfaktor, der mit einem Trägerprotein konjugiert ist, und ferner ein Adjuvans umfassend, wobei der humane autologe epidermale Wachstumsfaktor ein Antigen zum Auslösen der Autoimmunreaktion ist.

2. Impfstoffzusammensetzung nach Anspruch 1,
**dadurch gekennzeichnet, daß** sie einen rekombinanten humanen epidermalen Wachstumsfaktor enthält.

3. Impfstoffzusammensetzung nach Anspruch 1,
**dadurch gekennzeichnet, daß** sie die B-Kette von Choleratoxin als Trägerprotein enthält.

4. Impfstoffzusammensetzung nach Anspruch 1,
**dadurch gekennzeichnet, daß** sie ein Tetanus erzeugendes Toxoid als Trägerprotein enthält.

5. Impfstoffzusammensetzung nach Anspruch 1,
**dadurch gekennzeichnet, daß** sie einen monoklonalen Antikörper als Trägerprotein enthält.

6. Impfstoffzusammensetzung nach Anspruch 1,
**dadurch gekennzeichnet, daß** sie Protein der Außenmembran von Neisseria meningitidis als Trägerprotein enthält.

7. Impfstoffzusammensetzung nach Anspruch 1,
**dadurch gekennzeichnet, daß** sie Aluminiumhydroxid als Adjuvans enthält.

8. Impfstoffzusammensetzung nach einem der Ansprüche 1 bis 7 für die Behandlung einer vom epidermalen Wachstumsfaktor abhängigen malignen Erkrankung.

9. Verwendung eines autologen epidermalen Wachstumsfaktors eines Säugers bei der Herstellung eines Impfstoffs nach einem der Ansprüche 1 bis 7 für die Behandlung einer vom epidermalen Wachstumsfaktor abhängigen malignen Erkrankung.

10. Verwendung nach Anspruch 9,
**dadurch gekennzeichnet, daß** der Impfstoff für die Behandlung einer vom epidermalen Wachstumsfaktor abhängigen malignen Erkrankung beim Menschen dient.

## Revendications

1. Composition de vaccin pour produire une réponse auto-immune contre un facteur de croissance épidermique autologue, comprenant un facteur de croissance épidermique autologue humain conjugué à une protéine vectrice et comprenant en outre un adjuvant, ledit facteur de croissance épidermique autologue humain étant un antigène qui sert à déclencher ladite réponse auto-immune.

2. Composition de vaccin selon la revendication 1, **caractérisée en ce qu'**elle contient un facteur de croissance épidermique humain recombinant.

3. Composition de vaccin selon la revendication 1, **caractérisée en ce qu'**elle contient la chaîne B de la toxine cholérique comme protéine vectrice.

4. Composition de vaccin selon la revendication 1, **caractérisée en ce qu'**elle contient la toxoïde tétanique comme protéine vectrice.

5. Composition de vaccin selon la revendication 1, **caractérisée en ce qu'**elle contient un anticorps monoclonal comme protéine vectrice.

6. Composition de vaccin selon la revendication 1, **caractérisée en ce qu'**elle contient la protéine de membrane externe de Neisseria meningitidis comme protéine vectrice.

7. Composition de vaccin selon la revendication 1, **caractérisée en ce qu'**elle contient de l'hydroxyde d'aluminium comme adjuvant.

8. Composition de vaccin selon l'une quelconque des revendications 1-7 pour le traitement d'une maladie maligne dépendante du facteur de croissance épidermique.

9. Utilisation d'un facteur de croissance épidermique autologue d'un mammifère dans la préparation d'un vaccin selon l'une quelconque des revendications 1-7 pour le traitement d'une maladie maligne dépendante du facteur de croissance épidermique.

10. Utilisation selon la revendication 9, **caractérisée en ce que** le vaccin est destiné au traitement d'une maladie maligne dépendante du facteur de croissance épidermique chez les humains.
